# EUROPEAN PATENT APPLICATION

(11) **EP 1 239 044 A1**
(43) Date of publication of application: **11.09.2002**
(21) Application number: 01400593.8
(22) Date of filing: 07.03.2001
(51) Int. Cl.: C12N 15/82, A01H 1/06, A01H 1/00, A01H 5/10, C12N 15/29

(54) **Production of genetically modified plants with puroindolines**

(71) Applicant: Biogemma, 75001 Paris (FR)
(72) Inventor: Marion, Didier, 44300 Nantes (FR); Gautier, Marie-Françoise, 34090 Montpellier (FR); Joudrier, Philippe, 34070 Montpellier (FR); Messager, Arnaud, 63200 Riom (FR); Barsby, Tina, Cambridge CB4-OGZ (GB); Freeman, Judy, Cambridge CB4-OGZ (GB)
(74) Representative: Warcoin, Jacques

(57) **Abstract**

A new method for increasing expression of a puroindoline protein in a plant, said puroindoline protein modifying grain hardness, notably breadmaking quality, said method comprising the steps of:
(i) preparing a DNA construct which will allow the expression of a puroindoline protein,
(j) introducing said DNA construct into plant cell, plant tissue, or plant,
(k) selecting the plant cell, or plant tissue, or plant which stably maintain the puroindoline expression,
(l) and optionally regenerating fertile stable transformed plant.

SEQUENCE LISTING

<110> BIOGEMMA

<120> PRODUCTION OF GENETICALLY MODIFIED PLANTS WITH
      PUROINDOLINES

<130> D19435

<160> 2

<170> PatentIn Ver. 2.1

<210> 1
<211> 148
<212> PRT
<213> Triticum aestivum

<400> 1
Met Lys Thr Leu Phe Leu Leu Ala Leu Leu Ala Leu Val Ala Ser Thr
  1               5                  10                  15

Thr Phe Ala Gln Tyr Ser Glu Val Gly Gly Trp Tyr Asn Glu Val Gly
             20                  25                  30

Gly Gly Gly Gly Ser Gln Gln Cys Pro Gln Glu Arg Pro Lys Leu Ser
         35                  40                  45

Ser Cys Lys Asp Tyr Val Met Glu Arg Cys Phe Thr Met Lys Asp Phe
     50                  55                  60

Pro Val Thr Trp Pro Thr Lys Trp Trp Lys Gly Gly Cys Glu His Glu
 65                  70                  75                  80

Val Arg Glu Lys Cys Cys Lys Gln Leu Ser Gln Ile Ala Pro Gln Cys
                 85                  90                  95

Arg Cys Asp Ser Ile Arg Arg Val Ile Gln Gly Arg Leu Gly Gly Phe
            100                 105                 110

Leu Gly Ile Trp Arg Gly Glu Val Phe Lys Gln Leu Gln Arg Ala Gln
        115                 120                 125

Ser Leu Pro Ser Lys Cys Asn Met Gly Ala Asp Cys Lys Phe Pro Ser
    130                 135                 140

Gly Tyr Tyr Trp
145

<210> 2
<211> 148
<212> PRT
<213> Triticum aestivum

<400> 2
Met Lys Ala Leu Phe Leu Ile Gly Leu Leu Ala Leu Val Ala Ser Thr
  1               5                  10                  15

Ala Phe Ala Gln Tyr Ser Glu Val Val Gly Ser Tyr Asp Val Ala Gly

20                  25                  30

Gly Gly Gly Ala Gln Gln Cys Pro Val Glu Thr Lys Leu Asn Ser Cys
        35                  40                  45

Arg Asn Tyr Leu Leu Asp Arg Cys Ser Thr Met Lys Asp Phe Pro Val
    50                  55                  60

Thr Trp Arg Trp Trp Lys Trp Trp Lys Gly Gly Cys Gln Glu Leu Leu
65                  70                  75                  80

Gly Glu Cys Cys Ser Arg Leu Gly Gln Met Pro Pro Gln Cys Arg Cys
                85                  90                  95

Asn Ile Ile Gln Gly Ser Ile Gln Gly Asp Leu Gly Gly Ile Phe Gly
            100                 105                 110

Phe Gln Arg Asp Arg Ala Ser Lys Val Ile Gln Glu Ala Lys Asn Leu
        115                 120                 125

Pro Pro Arg Cys Asn Gln Gly Pro Pro Cys Asn Ile Pro Gly Thr Ile
    130                 135                 140

Gly Tyr Tyr Trp
145

## Description

The present invention relates to the use of genetic transformation to modify the properties of flours, notably breadmaking quality, by producing plants which overexpress puroindolines.

Wheat is one of the most important cereal crops in the world. Most of the wheat production is consumed as flour. Bread wheat accounts for about 80% of total consumption of wheat.

"Soft" and "hard" are the two main market classes of wheat (*Triticum aestivum L.*) and are distinguished by expression of the hardness gene. Although best known for its gluten-forming properties, the primary basis for discriminating different end uses in wheat is not protein content but grain hardness. Grain hardness refers to the texture of the kernel (caryopsis), that is, whether the endosperm is physically hard or soft. Nearly all of the world production and trade in wheat is identified as being either soft or hard. Generally speaking, hard wheat is used for bread whereas soft wheat is used for cookies, cakes, and pastries. The flour properties, notably breadmaking quality, are different according to the hardness or to the softness of the grain.

Friabilin and grain softness protein are two proteins isolated from wheat starch granules whose presence has been correlated with grain softness. Friabilins are marker proteins for grain softness. Puroindolines are low molecular weight basic cystine-rich proteins found in wheat seeds. Puroindolines show identity to the N-terminal sequence of friabilins.

Two highly conserved mutations in puroindoline-a and puroindoline-b have been found to be inseparably linked to grain hardness [Giroux M.J. and Morris C.F.(1998), Wheat grain hardness results from highly conserved mutations in the friabilin components puroindoline a and b. *Proc. Natl. Acad. Sci. USA,* 95(11) pp 6262-6266]. It was reported that the absence of puroindoline-a protein and transcript and a glycine-to-serine mutation in puroindoline-b are two highly conserved mutations associated with grain hardness and postulated "Elucidation of the molecular basis for grain hardness opens the way to understanding and eventually manipulating this wheat endosperm property".

The present invention discloses the modification of puroindoline-a or puroindoline-b protein expression by transformation of a plant. The objective was to modify puroindoline-a or puroindoline-b protein expression in a plant in order to modify the properties of flours, notably breadmaking quality.

The production of plants which overexpress puroindolines has never been reported. Such production of plants is realised by using genetic transformation.

Puroindoline-a and -b, have been isolated and characterized [Gautier M-F et al (1994) *Triticum aestivum* puroindolines, two basic cystine-rich seed proteins : cDNA sequence analysis and developmental gene expression. *Plant Molecular Biology* 25 : 43-57].

One INRA group (Institut National de la Recherche Agronomique, France) has demonstrated an inverse relationship between hardness and puroindoline content and shown that the breadmaking quality (*i.e.* the rheological properties of the dough and the structure of the bread crumb) of puroindoline-free hard cultivars can be drastically modified by the addition of puroindoline to the flours [Dubreil L. et al (1998) Effect of puroindolines on the breadmaking properties of wheat flour. *Cereal Chemistry* 75(2) :222-229].

The introduction of puroindoline-a or puroindoline-b cDNA into a plant has not previously been reported. The present invention aims to overcome one or more of the many problems in the art. For instance, experimental evidence included below demonstrates with embodiments of the present invention that plant transformation with puroindoline-a or puroindoline-b cDNA is achievable reproducibly.

According to a first aspect of the present invention, there is provided new methods and constructs for increasing expression of a puroindoline protein in a plant, said puroindoline protein modifying grain hardness, notably breadmaking quality, said method comprising the steps of:
(a) preparing a DNA construct which will allow the expression of a puroindoline protein,
(b) introducing said DNA construct into plant cell, plant tissue, or plant,
(c) selecting the plant cell, or plant tissue, or plant which stably maintain the puroindoline expression,
(d) and optionally regenerating fertile stable transformed plant.

According to an embodiment, said DNA construct is an expression cassette comprising a DNA of interest included between a promoter and a terminator. In a prefered embodiment, the DNA construct is a cDNA construct.

Said promoter is according to a prefered embodiment the Hight Molecular Weight Glutenin of wheat.

Said DNA of interest is puroindoline-a cDNA and/or puroindoline-b cDNA.

According to another aspect, the invention is related to a vector comprising an expression cassette as mentioned above.

According to another aspect, the invention is related to a host cell comprising said vector or a combination of said vectors.

According to another aspect, the invention is related to a plant cell transformed by said vector or combination of said vectors.

According to another aspect, the invention is related to a transgenic plant and progeny thereof wherein said plant is regenerated from said plant cell.

In a prefered embodiment, the plant and progeny thereof is chosen in the group consisting of wheat, maize, potato, barley and rice.

According to another aspect, the invention is related to a transgenic plant transformed with an expression cassette mentioned above, so that the puroindoline a or puroindoline b is expressed in the transgenic plant, said plant exhibiting a modified grain hardness compared to a progenitor plant which does not contain the expression cassette when the transgenic plant and the progenitor plant are cultivated under the same conditions.

According to another aspect, the invention is related to a seed, a progeny, a clone, cell line or cell of the said transgenic plant.

According to another aspect, the invention is related to the use of said transgenic plant or a part of it or a product of it, in order to prepare alimentary products or ingredients, and in particular in order to prepare flour.

According to another aspect, the invention is related to alimentary products or ingredients obtainable by this use.

The present invention provides new methods and constructs for producing plants with modified grain hardness, by introducing into these plants a DNA construct encoding a protein altering grain hardness.

A variety of proteins are capable of modifying grain hardness. The effect on grain hardness is linked to a variety of genes expressed in plants, including, for example, genes encoding friabilins, or genes encoding grain softness proteins, or genes encoding puroindoline-a or puroindoline-b. Such proteins allow grain softness, and thus improve breadmaking properties of wheat flour. Such proteins are equally capable of preventing foam destabilization by wheat polar lipids.

More particularly, the present invention provides new methods and constructs for producing plants with modified grain hardness, by introducing into these plants a DNA construct encoding a puroindoline-a or a puroindoline-b protein.

The term puroindoline refers to puroindoline-b corresponding to SEQ ID N°1 or puroindoline-a corresponding to SEQ ID N°2, but also to puroindoline homolog : any isolated puroindoline gene sequence exhibiting the same activity in terms of modification of grain texture to achieve may be used even if the DNA sequence is not exactly identical. Puroindoline cDNA or gene sequences present in the same species and/or homologs of the puroindoline gene present in other plant species can be identified and readily isolated without undue experimentation. The identification of homologs of puroindoline can be useful for developing plant model systems for purposes of discovering plant agonists or antagonists leading to larger or smaller altered physiology. Also cDNA libraries can be obtained from mRNA from plant cell lines or tissues known or suspected to express a puroindoline. Genomic DNA libraries can also be obtained. Besides, by performing polymerase chain reaction (PCR), genomic DNA or cDNA of puroindoline or homologs may be amplified for purpose of puroindoline DNA cloning or probes libraries.

According to the present invention, there is provided a method for increasing expression of a puroindoline protein in a plant, said puroindoline protein modifying grain hardness, notably breadmaking quality, said method comprising the steps of
(a) preparing a DNA construct which will allow the expression of a puroindoline protein,
(b) introducing said DNA construct into plant cell, plant tissue, or plant,
(c) selecting the plant cell, or plant tissue, or plant which stably maintain the puroindoline expression,
(d) and optionally regenerating fertile stable transformed plant.

### Target plants :

Notably, the plants useful for the invention are wheat, maize, potato, barley, and rice.

More particularly, plants are maize and wheat, specifically wheat.

The present invention concerns the modification of grain hardness, either by increasing it, or by decreasing it.

More particularly, the present invention concerns the improvement of breadmaking quality.

### Introduced DNA construct :

The preparation of the DNA construct may be varied, and the preferred embodiments will describe a number of features which the person of skill in the art will recognise as not being absolutely essential, but clearly advantageous. These include preparation methods of particular sequences to be introduced, certain features of the sequences and certain features of the associated vector, if any.

According to the invention, the DNA construct will include 5' and 3' regulatory sequences operably linked to the puroindoline gene. "Operably linked" refers to functional linkage between the 5' and 3' regulatory sequences and the controlled nucleic acid sequence.

The 5' regulatory sequence are notably promoters. These promoters would be selected for strength and/or spatial and temporal expression and/or inducibility. More particularly, the promoters are cell-specific, tissue-specific, organ-specific, development-specific, partially constitutive or fully constitutive (a promoter that strongly expresses in many or all plant tissues) or inducible promoters that can be expressed in a plant.
Transcription controlled by the operably linked promoter produces a functional messenger RNA whose transcription produces the puroindoline. An expression cassette will typically include the nucleic acid sequence for puroindoline operatly linked in the transformed plant to a transcription initiation region (a promoter sequence) and a transcription ending region.

Among the promoters useful for plant transformation within the framework of the present invention, the ones which can be used are:
- The 35S promoter of the cauliflower mosaic virus, or the 19S promoter or preferably the double constitutive 35S promoter, (pd 35S) described in the article of Kay et al. (1987).
- The pCRV promoter of the radish cruciferase gene (Depigny-This et al., 1992).
- The ubiquitin 1 promoter of maize (Christensen et al. 1996).
- The specific promoter of an organ or a development stage like the alpha tubulin promoter described in US 5.635.618.
- The histone promoters (EP 0 507 698).
- The HMWG promoter (Hight Molecular Weight Glutenin) of barley (Anderson O.D. et al., 1989) or of wheat (Roberts et al. 1989).
- The promoters induced by light; for example, the promoter of the gene of the small subunit of ribulose biphosphate carboxylase and of the chlorophyl a/b gene.
- The promoter of alcohol dehydrogenase of maize.
- The regulatory sequences of DNA-T of *Agrobacterium tumefaciens,* including mannopine synthase, nopaline synthase, octopine synthase.
- The promoter of rice actin followed by the rice actin intron (PAR-IAR) contained in the plasmid pAct+1-F4 described by McElroy et al. (1991).
- The promoters regulated during seed development such as the waxy, zein or bronze promoters of maize.
- The specific promoter N of a maize genomic clone, whose cDNA is referred to in the publication of Shen et al. (1994).
- The promoter regulatory sequence specific of regions or of particular plant tissues and more particularly of specific promoters of grains (Datla, R et al. 1997), especially the napin, phaseolin, glutenin, heliantin, albumin, oleosin ATS1 or ATS3 promoters, the pGEA1 and pGEA6 promoters corresponding to non coding region 5' of the reserve protein gene of the grain, GEA1 and GEA6 respectively of *Arabidopsis thaliana* (Gaubier et al., 1993).
- The promoter preferably chosen among the following ; the phenylalanine ammoniac lyase (PAL), HMG-CoA reductase (HMG), chitinase, glucanase, the proteinase inhibitor (PI), the genes of the PR1 family, vspB gene (US 5 670 349), the HMG2 (US 5 670 349), the beta galactosidase (Abgl) of apple or the amino cyclopropane carboxylate synthase promoter (ACC apple synthase) (WO 98/45445).
- The circovirus promoter (AU 689 311).

More particularly, the promoters which can be used are :
- The 35S promoter of the cauliflower mosaic virus, or the 19S promoter or preferably the double constitutive 35S promoter, (pd 35S) described in the article of Kay et al. (1987).
- The HMWG promoter (Hight Molecular Weight Glutenin) of barley (Anderson O.D. et al., 1989) or of wheat (Roberts et al. 1989).
- The regulatory sequences of DNA-T of *Agrobacterium tumefaciens*, including mannopine synthase, nopaline synthase, octopine synthase.
- The promoter of rice actin followed by the rice actin intron (PAR-IAR) contained in the plasmid pAct+1-F4 described by McElroy et al. (1991).
- The promoters regulated during seed development such as the waxy, zein or bronze promoters of maize.
- The promoter regulatory sequence specific of regions or of particular plant tissues and more particularly of specific promoters of grains (Datla, R et al. 1997), especially the napin, phaseolin, glutenin, heliantin, albumin, oleosin ATS1 or ATS3 promoters, the pGEA1 and pGEA6 promoters corresponding to non coding region 5' of the reserve protein gene of the grain, GEA1 and GEA6 respectively of *Arabidopsis thaliana* (Gaubier et al., 1993).

More specifically, the promoters which can be used are :
- The promoter regulatory sequence specific of regions or of particular plant tissues and more particularly of specific promoters of grains (Datla, R et al. 1997), especially the napin, phaseolin, glutenin, heliantin, albumin, oleosin ATS1 or ATS3 promoters, the pGEA1 and pGEA6 promoters corresponding to non coding region 5' of the reserve protein gene of the grain, GEA1 and GEA6 respectively of *Arabidopsis thaliana* (Gaubier et al., 1993).

According to a particularly preferred method of the invention, the promoter will be the high molecular weight glutenin promoter of wheat (HMWG), that is endosperm specific, for the expression cassette containing the gene encoding the puroindoline-a or the puroindoline-b.

In an additional embodiment of the present invention, the expression of the puroindoline may be engineered by increasing the copy number of the gene encoding the desired protein. One approach to producing a plant cell with increased copies of the desired gene is to transform with nucleic acid constructs that contain multiple copies of the gene. Alternatively, a gene encoding the desired polypeptide can be placed in a nucleic acid construct containing an amplification-selectable marker (ASM) gene such as the glutamine synthetase (GS) or dihydrofolate reductase gene. Cells transformed with such constructs are subjected to culturing regimes that select cell lines with increased copies of ASM gene. See Donn et al., 1984, J. Mol. Appl. Genet. 2:549-562, for a selection protocol used to isolate of a plant cell line containing amplified copies of the GS gene. Because the desired gene is closely linked to the ASM gene, cell lines that amplified the ASM gene would also likely to have amplified the gene encoding the desired growth modulation polypeptide.

The 3' regulatory sequence are notably terminators. These 3' terminator regions are linked to the gene of interest.

Among the terminators useful for plant transformation within the framework of the present invention, the ones which can be used are :
- The polyA 35S terminator of the cauliflower mosaic virus (CaMV), described in the article of Franck et al. (1980).
- The NOS terminator corresponding to the region in the non coding 3' region of the nopaline synthase gene of the plasmid Ti of *Agrobacterium tumefaciens* nopalin strain (Depicker et al. 1992).
- The histone terminator (EP 0 633 317).
- The tml terminator.

According to a particularly preferred method of the invention, the terminator will be the NOS terminator for the expression cassette containing the gene encoding the puroindoline-a or the puroindoline-b.

The expression cassettes may additionally contain transit peptide sequences in the expression cassette construct. There are numerous examples in the art of transit peptides which may be used to deliver a target protein into a plastid organelle such as the small subunit (SSU) transit peptide of ribulose biphosphate carboxylase.

Other elements like introns and enhancers can also be present in the nucleic sequence of interest in order to improve the expression of the gene of interest.

An example of an enhancer is the translation activator of tobacco mosaic virus (TEV) described by Carrington and Fred (1990).

Among useful introns, the first intron of maize adh1S can be placed between the promoter and the coding sequence. This intron when included in a gene construct increased the expression of the desired protein in maize cells (Callis et al., 1987). We also can use the 1^{st} intron of the shrunken 1 gene of the maize (Maas et al., 1991), the 1^{st} intron of the catalase gene of the bean catalase (CAT-1) (Ohta et al., 1990), the 2^{nd} intron of the ST-LS1 gene of potato (Vancanneyt et al. 1990), the DSV intron of the yellow dwarf virus of tobacco (Morris et al., 1992), the actin-1 intron (act-1) of rice (McElroy et al., 1990) and intron 1 of triosephosphate isomerase (TPI) (Snowdon et al., 1996).

The expression cassettes may additionally contain 5' leader sequences in the expression cassette construct. Such leader sequences can act to enhance translation. Such 5' leaders are known in the art and include, but *are* not limited to, picornavirus leaders, for example, the EMCV leader (Encephalomyocarditis 5' noncoding region) (Elroy-Stein, O., Fuerest, T.R., and Moss B. (1989) *PNAS USA,* 86 :6126-6130) ; potyvirus leaders, for example, the TEV leader (Tobacco etch Virus) (Allison et al. (1986) ; the MDMV leader (Maize Dwarf Mozaic Virus) *Virology,* 154 :9-20) ; the human immunoglobulin heavy-chain binding protein leader (BiP) (Macejack, D.G., and P. Sarnow (1991) *Nature,* 353 :90-94) ; the untranslated leader from the coat protein mRNA of alfalfa mosaic virus (AMV RNA 4) (Jobling, S.A., and Gehrke, L., (1987) *Nature,* 325 :622-625) ; the tobacco mosaic virus leader (TMV) (Gallie, D.R. et al. (1989) *Molecular Biology of RNA*, pages 237-256); and the maize chlorotic mottle virus leader (MCMV) (Lommel, S.A. et al. (1991) *Virology,* 81 :382-385). See also, Della-Cioppa et al. (1987) *Plant Physiology,* 84 :965-968. Other methods known to enhance translation can be utilized, for example introns, and the like.

In order to obtain the same modified plants, any other genes exhibiting homology or substantial homology with the puroindoline-a or puroindoline-b genes could be utilised as DNA sequence of interest.

The term "homology" refers to all nucleic sequences that are different from puroindoline-a or puroindoline-b gene sequence by substitution, deletion and/or insertion of nucleotides such that the nucleotide sequence encodes a protein with substantial homology to that encoded by the puroindoline-a or puroindoline-b gene sequences.

In an embodiment the DNA sequence of interest is chosen among :
- (a) the DNA sequences described by Gautier (1994), that encode the amino acid sequences SEQ ID N°1 corresponding to puroindoline-b and SEQ ID N°2 corresponding to puroindoline-a,
- (b) any nucleotide sequence homologous to the sequences of (a),
- (c) any nucleotide sequence complementary of sequence of (a) or (b),
- (d) any nucleotide sequence of a fragment representative of a sequence of (a) or (b) or (c),
- (e) any nucleotide sequence that can be obtained from a sequence of (a) or (b) or (c) or (d)
- (f) any nucleotide sequence of degenerate variants of sequence (a) to (e)

Preferably such a homologous nucleic acid sequence is at least 75% identical to the puroindoline-a or puroindoline-b gene sequence, preferably at least 85% identical, more preferably at least 90,95,98 % identical.

A percentage of identity between two nucleic sequences refers to the percentage of nucletotides identical between the two nucleic sequences, these sequences being optimally aligned. The percentage is statistical. The optimal alignment for the comparaison may be obtained for instance by the algorithm of local homology of Smith and Waterman (1981), or of Neddleman and Wunsch (1970), or by softwares using these algorithms (GAP, BESTFIT, BLAST).

In a preferential manner such a homologous nucleic acid sequence hybridizes to the complementary sequence of the puroindoline-a or puroindoline-b gene sequence under stringent conditions. The parameters defining the stringency conditions depend on the temperature in which 50% of the coupled strands separate (Tm).

Concerning the sequences comprising more than 30 bases, Tm is defined by the relation Tm = 81.5 + 0.41 (%G+C) + 16.6 Log (concentration in cations)- 0.63 (%fornamide) - (600/numbers of bases). (Sambrook et al.(1989), *Molecular Cloning, A Laboratory Manual*, *Cold Spring Harbour Laboratory Press,* p. 9.54-9.62).

Concerning the sequences less long than 30 bases, Tm is defined by the relation Tm = 4(G+C) + 2(A+T).

In appropriate stringency conditions, in which non-specific sequences do not hybridize, the temperature of hybridization is approximately between 5 and 30°C, preferable between 5 and 10°C under Tm and hybridization buffers used are preferably solutions of higher ionic force like a solution 6*SSC for example.

Representative fragments include in particular fragments that can be used as primers or probes in methods allowing to obtain or identify such homologous sequences, in particular by using PCR technics for instance described in US 4 683 202. PCR like technics may also be used, for instance RT (reverse transcriptase) PCR, SDA (Strand Displacement Amplification - Walker et al., 1992), TAS (Tanscription based Amplification System - Kwoh et al. 1989). Representative fragments also include antisens nucleic sequences, able to inhibate the expression of the puroindoline-a or puroindoline-b by hybridation with a target sequence corresponding to puroindoline-a or puroindoline-b.

A variant sequence refers to a sequence existing naturally, modified by substitution, deletion and/or insertion of nucleotide(s) in the sequence.

Complementary sequences refer to DNA sequences complementary of that of a) or b) and which orientation is inversed.

The cassette may additionally contain at least one gene to be cotransformed into the organism. Alternatively, the additional gene(s) of interest can be provided on another expression cassette.

According to the invention, the DNA construct may contain one or several selectable markers useful for transformation and selection. These selectable markers include, but are not limited to, antibiotic resistance, herbicide resistance or visual observation. Other phenotypic markers are known in the art and may be used in this invention.

A number of selective agents and resistance genes are known in the art. (See, for example, Hauptmann et al. (1988) *Plant Physiol.* 86 :602-606 ; Dekeyser et al. (1988) *Plant Physiol.* 90 :217-223 ; Eichholtz et al. (1987) *Somatic Cell and Molecular Genetics* 13 :67-76 ; and Meijer et al. (1991) *Plant Molecular Biology* 16 :807-820.

Notably the selectable marker used can be :
- The sulfonamide herbicide Asulam resistance gene, sul (described in WO 98/49316) encoding a type I dihydropterate synthase (DHPS) as a selectable marker in monocotyledone species (such as wheat).
- The nptII gene conferring resistance to kanamycin (Bevan et al. (1983), *Nature* 304 :184-187).
- The hph gene conferring resistance to hygromycin (Gritz et al (1983), *Gene* 25 :179-188).
- The bar gene conferring tolerance to bialaphos (White et al. (1990), NAR 18 :1062).
- The EPSPS gene conferring tolerance to glyophosate (US 5,188,642).
- The HPPD gene conferring tolerance to isoxazoles (WO 96/38567).
- The gene encoding for the GUS enzyme.
- The genes encoding for neomycin phosphotransferase confers to transformed cells resistance to a group of antibiotics including kanamycin, paromomycin and neomycin.
- The green fluorescent protein (GFP), expression of which, confers a recognizible physical characteristic to transformed cells. When GFP-transformed cells are visualised under UV or a blue light they emit a characteristic green light which can be observed using appropriate equipment.
- The chloramphenicol transferase gene, expression of which, detoxifies chloramphenicol.
- Inhibitors such as amino-glycoside antibiotics which interfere with the translation machinery of prokaryotic and eukaryotic cells, may be utilized. Such inhibitors include kanamycin, G418, hygromycin, etc. Such inhibitors can be inactivated by phosphorylation reactions mediated by the products of either the Tn 5 neomycin phosphotransferase II (npt-II) gene or the hygromycin B resistance gene from E. coli. (See, for example, Herrera-Estrella et al. (1983) *EMBO* J2 :987-995 ; Waldron et al. (1985) *Plant Mol Biol* 5 :103-108 ; and the references cited therein.).

According to a particularly preferred method of the invention, the marker is the sulfonamide herbicide Asulam resistance gene.

In the same way, for the sulfonamide herbicide Asulam resistance expression cassette, we can use different regulatory elements as described before, notably promoters, terminators, and transit peptides.

According to a particularly preferred method of the invention, the promoter will be the rice actin 1 promoter, for the expression cassette containing the sulfonamide herbicide Asulam resistance gene.

According to a particularly preferred method of the invention, the transit peptide sequence contained in the sulfonamide herbicide Asulam resistance gene expression cassette will be a chloroplast targetting sequence.

In preparing the expression cassettes, the various DNA fragments may be manipulated, so as to provide DNA sequences in the proper orientation and, as approppriate, in the proper reading frame. Towards this end, adapters or linkers may be employed to join the DNA fragments and/or other manipulations may be required to provide convenient restriction sites, removal of superflous DNA, removal of restriction sites, or the like. For this purpose, in vitro mutagenesis, primer repair, restriction, annealing, ligation, PCR, or the like may be employed, where nucleotide insertions, deletions or substitutions, for example transitions and transversions, may be involved.

### Transformation :

The DNA construct which will allow the expression of a puroindoline protein is introduced into a plant cell, a plant tissue, or a plant.

The transformation of plants in accordance with the invention may be carried out in essentially any of the various ways known to those skilled in the art of plant molecular biology. See, in general, *Methods in Enzymology* Vol. 153 ("Recombinant DNA Part D") 1987, Wu and Grossman Eds., Academic Press, incorporated herein by reference.

As used herein, the term "transformation" encompasses the genetic manipulation of the plant, cell, cell line, callus, tissue, plant part, and the like. That is, such cell, cell line, tissue, plant part, or plant which has been altered by the presence of recombinant DNA wherein said DNA is introduced into the genetic material within the cell, either chromosomally, or extra-chromosomally. Recombinant DNA includes foreign DNA, heterologous DNA, and chimeric DNA. The recombinant DNA can be random either targeted in a specific locus by homologous recombination accordin to technics already known by the one skilled in the art.

In one embodiment, the foreign nucleic acid is mechanically transferred by microinjection directly into plant cells (cytoplasm or nucleus) by use of micropipettes.

Alternatively, the foreign nucleic acid may be transferred into the plant cell by using polyethylene glycol in presence of bivalent cations (Ca²⁺). This forms a precipitation complex with the genetic material that is taken up by the cell. (Paszkowski et al., (1984) *EMBO J.* 3 :2717-22).

In an other embodiment of the invention, the introduced gene may be introduced into the plant cells by electroporation. (Fromm et al., (1985) "Expression of Genes Transferred into Monocot and Dicot Plant Cells by Electroporation," *Proc. Natl Acad. Sci. USA* 82 :5824, which is incorporated herein by reference). In this technique, plant protoplasts are electroporated in the presence of plasmids or nucleic acids containing the relevant genetic construct. Electrical impulses of high field strength reversibly permeabilize biomembranes allowing the introduction of the plasmids. Electroporated plant protoplasts reform the cell wall, divide, and form a plant callus. Selection of the transformed plant cells with the transformed gene can be accomplished using phenotypic markers.

Cauliflower mosaic virus (CaMV) may also be used as a vector for introducing the foreign nucleic acid into plant cells. (Hohn et al., (1982) "Molecular Biology of Plant Tumors,"Academic Press, New York, pp.549-560 ; Howell, United States Patent No. 4,407,956). CaMV viral DNA genome is inserted into a parent bacterial plasmid creating a recombinant DNA molecule which can be propagated in bacteria. After cloning, the recombinant plasmid again may be cloned and further modified by introduction of the desired DNA sequence into the unique restriction site of the linker. The modified viral portion of the recombinant plasmid is then excised from the parent bacterial plasmid, and used to inoculate the plant cells or plants.

Another method of introduction of nucleic acid segments into plant cells is to infect a plant cell, an explant, a meristem or a seed with *Agrobacterium tumefaciens* transformed with the segment. Under appropriate conditions known in the art, the transformed plant cells are grown to form shoots, roots, and develop further into plants. The nucleic acid segments can be introduced into appropriate plant cells, for example, by means of the Ti plasmid of *Agrobacterium tumefaciens.* The Ti plasmid is transmitted to plant cells upon infection by *Agrobacterium tumefaciens*, and is stably integrated into the plant genome. (Horsch et al., (1984) "Inheritance of Functional Foreign Genes in Plants," *Science,* 233 :496-498 ; Fraley et al., (1983) *Proc. Natl. Acad. Sci. USA* 80 :4803).

Further wheat tissues that are capable of transformation according to the methods described herein include calli, cell suspension cultures, anthers, microspores, embryos, inflorescences, and the like. Cell suspension cultures can be derived from calli of embryos, leaf tissues, young inflorescences, anthers, etc.

Callus can be originated from any tissues of wheat plants including *Triticum aestivum* and *Triticum durum.* Preferably the tissue utilized in initiating callus is immature tissue such as immature embryos, immature inflorescences, and the basal portion of young leaves.

Alternatively, callus can be originated from anthers, microspores, mature embryos and in principle any other tissue of wheat capable of forming callus and or secondary embryos.

An especially useful tissue for producing regenerable callus is the scutellum of immature wheat embryos.

A preferred method of introducing the nucleic acid segments into plant cells is transformation by particle bombardment. The plant cells or tissues, preferably immature embryos, to be transformed are bombarded with a particle bombardment device. Particles are coated with DNA sequences of interest. Particle bombardment offers a rapid method for transformation. Generally the plant cells or tissues, preferably immature embryos are shot at least one time. However, multiple shots of the plant cells or tissues, preferably immature embryos, may be performed to enhance transformation frequency. See WO 98/49316 incorporated herein by reference.

After transformation of the plant cells or plant, those plant cells or plant transformed by particle bombardment so that the desired DNA segment is integrated can be selected by an appropriate phenotypic marker.

In the present invention, a cotransformation system was used whereby the selectable marker gene, the sulfonamide herbicide Asulam resistance gene, and the gene of interest, puroindoline-a or puroindoline-b, were introduced on two separate expression cassettes.

The production of plant cells with a reduced activity of puroindoline may also be achieved by using the DNA molecules mentioned. Possibilities are the expression of a corresponding antisense-RNA to reduce the activity of puroindoline in plant cells.

### Selection :

Selection of transgenotes for further study will typically based upon a medium selection. The term "transgenote" refers to the immediate product of the transformation process and to resultant whole transgenic plants.

In an embodiment of the present invention, the engineered plant material is selected or screened for transformants (those that have incorporated or integrated the introduced gene construct(s)). An isolated transformant may then be regenerated into a plant. Alternatively, the engineered plant material may be regenerated into a plant or plantlet before subjecting the derived plant or plantlet to selection or screening for the marker gene traits. Procedures for regenerating plants from plant cells, tissues or organs, either before or after selecting or screening for marker gene(s), are known to those skilled in the art.

A transformed plant cell, callus, tissue or plant may be identified and isolated by selecting or screening the engineered plant material for traits encoded by the marker genes present on the transforming DNA. For instance, selection may be performed by growing the engineered plant material on media containing inhibitory amount of the antibiotic or herbicide to which the transforming gene construct confers resistance. Further, transformed plants and plant cells may also be identified by screening for the activities of any visible marker genes (e.g., the .beta.-glucuronidase, luciferase, B or C1 genes) that may be present on the recombinant nucleic acid constructs of the present invention. Such selection and screening methodologies are well known to those skilled in the art.

Medium comprising Asulam is the preferred medium for the selection step involved in growing transformed plant cells or tissues, preferably immature embryos.

After growth on selection medium the transformed and selected tissue is allowed to grow.

For plants surviving selection on Asulam-containing medium, genomic DNA is extracted and probed to confirm transformation. Methods are available in the art for the isolation of DNA from biological material in culture as well as for confirming the presence of DNA of interest. Such methods to confirm include
1) PCR analysis as well as Southern blot hybridisation for determining the structure of the recombinant DNA insert. See, Southern, EM (1975) Journal of Molecular Biology 98 :503 and Mullis, KB (1987) Methods in Enzymology 155 :335 ;
2) Northern blot, reverse transcriptase-PCR amplification for detecting and examining RNA transcripts of the gene construct;
3) Protein gel electrophoresis, Western blot techniques, immunoprecipitation, or enzyme-linked immunoassays, where the gene construct products are proteins.

### Protein expression :

The protein expression, preferably puroindoline-a or puroindoline-b expression, is analysed by Enzyme-Linked-Immunoabsorbant-Assay (ELISA known by the one skilled in the art) in transgenic plants.

### Regeneration :

Normally, regeneration will be involved in obtaining a whole plant from the transformation process. The term "regeneration" as used herein, means growing a whole plant cell, a group of plant cells, a plant part or a plant piece (for example, from a protoplast, callus, or tissue part).

Methods of regenerating whole plants from plant cells are known in the art, and the method of obtaining transformed and regenerated plants is not critical to this invention.

In general, transformed plant cells are cultured in an appropriate medium, which may contain selective agents such as antibiotics, where selectable markers are used to facilitate identification, of transformed plant cells. Once callus forms, shoot formation can be encouraged by employing appropriate plant hormones in accordance with known methods and shoots transferred to rooting medium for regeneration of plants. The plants may then be used to establish repetitive generations, either from seeds or using vegetative propagation techniques.

Thus, the invention also relates to plant tissue, plants or seeds containing the nucleic acid sequences described above. In the context of the disclosure "plant tissue" refers to any tissue of a plant, in planta, or in culture. This term includes, but is not limited to, whole plants, plant cells, plant organs, plant seeds, protoplasts, callus, cell cultures and any groups of plant cells organized into structural and/or functional units. The use of this term in cunjunction with, or in the absence of, any specific type of plant tissue as listed above or otherwise embraced by this definition is not limited to be exclusive of any other type of plant tissue.

The cells which have been transformed may be grown into plants in accordance with conventional techniques. See for example, McCormick et al. (1986) *Plant Cell Reports*, 5 :81-84. These plants may then be grown, and either pollinated with the same transformed strain or different strains, and the resulting hybrid having the desired phenotypic characteristic identified. Two or more generations may be grown to ensure that the introduced phenotypic characteristic is stably maintained and inherited and then seeds harvested to ensure the desired phenotype or other property has been achieved.

The present invention thus encompasses the fertile transgenic plants and transformed seeds thereof, as well as the subsequent progeny and products derived therefrom.

Parts obtained from the regenerated plant, such as flowers, seeds, leaves, branches, fruit, and the like are covered by the invention, provided that these parts comprise cells which have been so transformed.

Progeny and variants, and mutants of the regenerated plants are also included within the scope of this invention, provided that these parts comprise the introduced DNA sequences.

Also considered as a specific part of the invention are transgenic wheat plants containing an expression cassette according to the present invention.

Improved embryogenic cultures of wheat can be obtained by using previously regenerated material as a source of starting material. The starting material for these improved cultures may be either immature embryos obtained directly from regenerated plants, or the starting material may be seeds from regenerated plants grown as as source of immature embryos.

### Vectors :

The method refers to the vectors used for carrying out the invention.

The decision as to whether to use a vector, or which vector to use, will be guided by the method of transformation selected, and by the host cell selected.

In the following example, a naked nucleic acid introduction method is used. Then the vector need be no more than the minimal nucleic acid sequences necessary to confer the desired phenotype, without the need for additional sequences.

Possible vectors include the Ti plasmid vectors, shuttle vectors designed merely to maximally yield high numbers of copies, episomal vectors containing minimal sequences necessary for ultimate replication once transformation has occured, transposon vectors, including the possibility of RNA forms of the gene sequences. The selection of vectors and methods to construct them are commonly known to persons of ordinary skill in the art and are described in general technical references (Mullis, KB (1987),*Methods in Enzymology*).

For other transformation methods requiring a vector, selection of an appropriate vector is relatively simple, as the constraints are minimal. The apparent minimal traits of the vector are that the desired nucleic acid sequence be introduced in a relatively intact state. Thus, any vector which will produce a plant carrying the introduced DNA sequence should be sufficient. Also, any vector which will introduce a substantially intact RNA which can ultimately be converted into a stably maintained DNA sequence should be acceptable.

However, any additional attached vector sequences which will confer resistance to degradation of the nucleic acid fragment to be introduced, which assists in the process of genomic integration or provides a means to easily select for those cells or plants which are actually, in fact, transformed are advantageous and greatly decrease the difficulty of selecting useable transgenotes.

The vector can exist, for example, in the form of a phage, a plasmid or a cosmid. The construction of such expression vectors for transformation is well known in the art and uses standard techniques. We can mention especially the methods described by Sambrook et al. (1989).

### Host Cells :

The method refers to the host cells used for carrying out the invention.

The decision as to whether to use a host cell, or which host cell to use, will be guided by the method of transformation.

In a particular embodiment, the present invention provides a host cell comprising a vector as described above. Any of a large number of available and well-known host cells may be used in the practice of this invention. The selection of a particular host is dependent upon a number of factors recognized by the art. These include, for example, compatibility with the chosen expression vector, bio-safety and costs. Useful hosts include bacteria such as E. *coli sp.* or *Agrobacterium.*

More particularly, the host cell used in carrying out the invention is *Agrobacterium tumefaciens*, according to the method described in the article of An et al., 1986, or *Agrobacterium rhizogenes,* according to the method described in the article of Jouanin et al., 1987.

### EXAMPLES

By way of example, and not limitation, an examplary preferred embodiment of the present invention entails introducing a full-length puroindoline-a or puroindoline-b coding sequence in sense orientation which would be operably linked to a high molecular weight glutenin promoter of wheat and to a Nos terminator into wheat immature embryos. These transgenotes are grown into plants and variations in grain hardness are observed.

### EXAMPLE 1: Transformation of wheat with puroindoline genes

Immature embryos of the spring wheat line NB 1 (obtained from Nickerson UK Ltd, Rothwell, Lincs.) were transformed by particle bombardment using resistance to the sulfonamide herbicide Asulam as the selectable marker. See WO 98/49316 which is incorporated here by reference.

A cotransformation system was used whereby the selectable marker gene, the sulfonamide herbicide Asulam resistance gene, and the gene of interest, puroindoline-a or puroindoline-b [Gautier M-F et al. (1994) *Triticum aestivum* puroindolines, two basic cystine-rich seed proteins : cDNA sequence analysis and developmental gene expression. *Plant Molecular Biology* 25 : 43-57], were introduced on separate DNA constructs. Furthermore, a "clean transformation" system was employed whereby the expression cassettes containing the genes were excised from their respective plasmids prior to bombardment in order to prevent the introduction of unwanted bacterial sequences from the plasmids into the plant genome.

### a) Construct preparation

### Selectable marker

The sulfonamide herbicide Asulam resistance expression cassette was excised from plasmid pWP258 (WO 98/49316) using the restriction enzymes XbaI and XhoI to give a fragment of 3410 base pairs comprising the sulfonamide herbicide Asulam resistance gene fused to a chloroplast targetting sequence and under the control of the rice actin 1 promoter.

### Gene of interest

The promoter of the high molecular weight glutenin (HMWG) gene 1Dx5 [Halford, N. *et al.* (1989) Functional analysis of the upstream regions of a silent and a expressed member of a family of wheat seed protein genes in transgenic tobacco. *Plant Science* 62 :207-216] was isolated from wheat genomic deoxyribonucleotidic acid (DNA) using Polymerase Chain Reaction (PCR). Primers carried ClaI and EcoRI sites. The HMWG promoter was digested with ClaI-EcoRI and ligated into the ClaI-EcoRI site of pBluescript® (Stratagene).

The Nos terminator (Depicker et al. 1992) was isolated as a PCR fragment and ligated into the BamHI-SacI site of this subclone to make pDV03000 (WO 00/31274).

The cDNA sequences pTa31 and pTa19B2 [Gautier M-F et al. (1994) *Triticum aestivum* puroindolines, two basic cystine-rich seed proteins : cDNA sequence analysis and developmental gene expression. *Plant Molecular Biology* 25 : 43-57 incorporated herein by reference] encoding precursors of puroindoline-a and puroindoline-b respectively having cleavable N-terminal and C-terminal sequences putatively involved in targeting, were introduced between the HMWG promoter and the Nos terminator of pDV03000 as follows.

The cDNA clones were digested with NotI and SalI and the ends made blunt. The blunt-ended pTa31 and pTA19B2 were ligated into the SmaI site of pDV03000 to give pPIA and pPIB, respectively.

The sequence TCGACCCACGCGTCCG was introduced between the SmaI site and the beginning of the puroindoline-a or the puroindoline-b cDNA sequences. Using this cloning strategy no ATG was introduced between the HMWG promoter and the ATG of puroindoline-a or the puroindoline-b sequences.

The sequence GCGGCC was introduced at the end of the puroindoline-a or the puroindoline-b cDNA sequences between the poly(A) tail and the SmaI site.

The puroindoline-a expression cassette, PIA, containing the HMWG promoter, the puroindoline-a gene, and the Nos terminator, was removed from pPIA within a fragment of approximately 2kb using a PvuII digest. The puroindoline-b expression cassette, PIB, containing the HMWG promoter, the puroindoline-b gene, and the Nos terminator was removed from pPIB within a fragment of approximately 1.7 kb using a SalI + SstI digest.

### b) Transformation

Immature embryos of the spring wheat line NB1 were transformed by particle bombardment as described in WO 98/49316 with a mixture of the sulfonamide herbicide Asulam resistance expression cassette from pWP258 plus either the PIA or the PIB. The ratio gene of interest expression cassette (PIA or PIB) : sulfonamide herbicide Asulam resistance expression cassette was 1:1 in each case.

### c) Molecular analysis of regenerants

Plants surviving selection on Asulam-containing medium were tested for the presence of the puroindoline-a or puroindoline-b cDNA by PCR. Genomic DNA was extracted using the method of Edwards *et al.* [(1991) *Nucleic Acids Research* Vol. 19 No. 6 p. 1349] and resuspended in 50µl of TE buffer, which composition is well known in the art.

PCR reactions were performed using primers designed to amplify a 269bp product in the presence of puroindoline-a cDNA: 5' CTGTCACATACCACTGCGTGTG 3' and 5' TCATCGCAAGACCGGCAACAGG 3' or a 580bp product in the presence of puroindoline-b cDNA:

5’ CGCCGATTACGTGGCTTTAGCA 3’ and

5' GCTGCTTGCAGCACTTCTCCCG 3'.

Each 50µl PCR reaction contained 1xPCR buffer as supplied by the manufacturer (GibcoBRL), 2mM MgCl, 0.2mM each of dATP, dCTP, dGTP and dTTP, 0.2pmoles each of forward and reverse primers (added at "hot start"), and 1µl of sample DNA. The reaction conditions were as follows: "hot start" (94°C, 3min) followed by 30 cycles of denaturation (95°C, 30sec), annealing (60°C, 30sec) and extension (73°C, 2min) followed by 1 cycle of 73°C (5min) and then held at 24°C.

Plants which tested positive by PCR were further analysed by Southern blot hybridisation. Southern blot analysis was performed on DNA from a full scale (9ml) extraction from lyophilized ground tissue [Stacey and Isaac, (1994) *Methods in Molecular Biology,* Vol. 28 : Protocols for nucleic acid analysis by nonradioactive probes, pp9-15, Edited by P. G. Isaac, Humana Press Inc., Totawa, NJ]. Puroindoline-a DNA samples were diluted to 0.2mg/ml and digested with the restriction enzyme KpnI; puroindoline-b DNA samples were digested with ClaI. Restriction enzyme digestion, gel electrophoresis and vacuum blotting were carried out as described by Stacey and Isaac (1994). Digoxygenin-labelled puroindoline-a and puroindoline-b probes were produced by PCR according to the method of McCreery and Helentjaris [(1994a) *Methods in Molecular Biology,* Vol. 28 : Protocols for nucleic acid analysis by non-radioactive probes, pp67-71, Edited by P. G. Isaac, Humana Press Inc., Totawa, NJ]. The primers described above were used to label the respective probes. Hybridisation of the probe to the Southern blot and detection by chemiluminescence was performed according to the method of McCreery and Helentjaris [(1994b) *Methods in Molecular Biology,* Vol. 28 : Protocols for nucleic acid analysis by non-radioactive probes, pp107-112, Edited by P. G. Isaac, Humana Press Inc., Totawa, NJ].

### d) Puroindoline expression in transgenic wheat

Puroindoline-a transformants, tPIA, and puroindoline-b transformants, tPIB, identified by PCR were grown to maturity and the seed harvested. Expression of puroidoline-a and puroindoline-b in the T₁ seed was measured by Enzyme-Linked Immunosorbent Assay (ELISA) ( [INRA method]. Expression of puroindoline-a in the tPIA lines ranged from x1.25 to x8 relative to the control. Expression of puroindoline-b in the tPIB lines ranged from x1 (*i.e.* no significant increase) to x4 relative to the control.

The tPIA line 258.1x was found to have five times more puroindoline-a than control. The level of puroindoline-b was not modified.

The tPIB lines 219.1a, 219.1d and 219.1g were found to have 2.5 to 3 times more puroindoline-b than control seeds (Table 1). The level of puroindoline-a was not modified.

**Table 1:**

| Expression of puroindoline-b in transgenic plants | |
|---|---|
| **Seed** | **% of Total Protein** |
| control | 0.026 |
| 219.1a | 0.074 |
| 219.1d | 0.066 |
| 219.1g | 0.076 |

### e) Southern characterisation

Lines 258.1x (with elevated puroindoline-a) and 219.1g (with elevated puroindoline-b) were selected to be further advanced. Southern analysis indicated that 258.1x had 8 or more inserts and 219. 1g had 10 or more inserts.

### f) Production and multiplication of homozygous seed lots

Lines 258.1x and 219.1g were selected for progression to homozygosity and multiplication. T₁ and T₂ populations were grown up and transgenic and non-transgenic segregants distinguished by PCR, as described above. When all T₂ plants derived from an individual were transgenic they were further characterised by Southern. Where the integration patterns were identical the corresponding T₁ segregant was regarded as homozygous. T₃ seed was harvested from homozygous plants.

Homozygous T₃ seed and non-transgenic segregants (controls) of lines 258.1x and 219.1g were analysed by ELISA as described previously to determine the level of puroindoline-a and -b. The results are shown below in Tables 2 and 3. The increased level of the relevant puroindoline was observed in the homozygous T₃ seed. The level of puroindoline-a was further elevated, eightfold, in one segregant of line 258.1x.

**Table 2.**

| Expression of puroindoline-a in T₃ seed of tPIA line 258.1x | |
|---|---|
| **Seed of :** | **Puroindoline-a** **(% of total protein)** |
| 258.1x/3/2 non transgenic segregant | 0.081 |
| 258.1x/16/26 homozygous transgenic | 0.640 |
| 258.1x/23/28 homozygous transgenic | 0.414 |

The level of puroindoline-b in these seeds was not modified.

**Table 3.**

| Expression of puroindoline-b in T₃ seed of tPIB line 219.1g | |
|---|---|
| **Seed of :** | **Puroindoline-b** **(% of total protein)** |
| 219.1g/8/28 non-transgenic segregant | 0.037 |
| 219.1g/14/22 homozygous transgenic | 0.070 |
| 219.1g/19/29 homozygous transgenic | 0.087 |
| 219.1g/21/14 homozygous transgenic | 0.124 |

The level of puroindoline-a in these seeds was not modified.

Homozygous T3 seed and non-transgenic segregants were further multiplied in the field in order to produce sufficient material for functionality testing.

### g) Crossing of transgenic lines

i) Crossing of transgenic puroindoline-a with transgenic puroindoline-b line.
   Homozygous segregants of transgenic lines 258.1x and 219.1g were crossed. A simultaneous increase of puroindoline-a and puroindoline-b was observed in the progeny. Further crosses were made between lines having high levels of expression of each puroindoline as homozygous progeny were identified.
ii) Crossing of transgenic puroindoline-a and puroindoline-b lines into alternative backgrounds
   Homozygous segregants of 258.1x and 219.1g were crossed into three different commercial wheat types : high quality bread wheat (ULI 3, Glenlea, F.aurore), biscuit type (97-2149 and 97-0118) and an ingredient wheat (hypersoft, Bucan, 1159).

### EXAMPLE 2 : Method for making a sandwich loaf with flour high in puroindolines produced by transgenic wheat overexpressing puroindolines.

An embodiment of the present invention entails enhancing the breadmaking quality of sandwich loaf.

### a) Manufacturing process

The ingredients used and the manufacturing process are summarised below.

### Ingredients :

**Table 4.**

| List of ingredients used for breadmaking | | | |
|---|---|---|---|
| | **Control** | **Test 1** | **Test 2** |
| Flour T55 | 100% | | |
| Flour high in puroindolines | | 1% | 2% |
| Water | (suitable moisture : about 57%) | | |
| Salt | 2% | 2% | 2% |
| Leaven (yeast) | 5% | 5% | 5% |
| Lipids | 4% | 4% | 4% |
| Sugar | 4% | 4% | 4% |
| Propionate | 0,5% | 0,5% | 0,5% |

### Process :

A spiral-kneading machine was used.
Step 1 : "frasage" at speed 1 for 5 minutes
Step 2 : kneading at speed 2 for 10 minutes
Step 3 : rest for 5 minutes
Step 4 : the dough was divided into 400g balls for closed moulds and 430 g balls for open moulds
Step 5 : 15 minutes of expansion
Step 6 : 75 minutes of finishing at 30°C
Step 7 : Cooking for 30 minutes at 180°C
Step 8 : Sweating for 2 hours at room temperature
Step 9 : packaging in plastic bags
Step 10 : storage at 20°C

### b) Evaluation of dough quality

The dough quality was manually evaluated according to the tackiness, the consistency, the extensibility and the elasticity of the dough.

### c) Evaluation of sandwich loaves

### Sandwich loaves cooked in open moulds

The appearance of the bread was evaluated according to the colour and to the *corseting*. A light (weak) corseting was required. The volume of two breads per test was measured with a volumeter.

### Sandwich loaves cooked in closed moulds

Loaves were sliced just before the quality experiments.

### i) Evaluation on the same day

The crumb was evaluated after the sweating step on a stack of 5 slices according to suppleness, elasticity, and tackiness when pressed with the fingers, and the crumb relaxation.

The suppleness corresponded to the crumb capacity to compress.

The elasticity corresponded to the speed and the intensity of the crumb coming back to the initial form after compression.

The tackiness was the evaluation of the crumb adherence to fingers.

### ii) Evaluation after 7, 14 and 21 days of storage

Measurement by texturometer completed the evaluation of manually assessed characters. The measures were carried out 5 times per bread on a stack of 2 slices after 7, 14 and 21 days of storage.

### -Evaluation of alveolus quantity

A slice of sandwich loaf was photocopied in order to evaluate the quantity of air in crumb.

### -Sensory evaluation

The taste and the change in flavour were estimated on the fresh product and after storage.

Sandwich loaves cooked with flour high in puroindolines have better taste than sandwich loaves prepared with normal flour.

### EXAMPLE 3 : Use of flour high in puroindolines in an emulsified sauce : light mayonnaise.

An embodiment of the present invention entails enhancing the quality of a light emulsified sauce (mayonnaise).

### a) Manufacturing process

The ingredients used and the manufacturing process are summarised below.

### Ingredients :

**Table 5.**

| List of ingredients used for making mayonnaise | | |
|---|---|---|
| | **Control** | **Test** |
| Flour T55 | 1,2% | |
| Flour high in puroindolines | | 1,2% |
| Salt | 1,2% | 1,2% |
| Dextrose | 3% | 3% |
| Egg yolk powder | 5% | 5% |
| Vinegar | 7% | 7% |
| Mustard | 7% | 7% |
| Sunflower oil | 47% | 47% |
| Water | 28,6% | 28,6% |

Water can be used in place of mustard in order to decrease the mayonnaise stability.

### Process :

Flour, salt, dextrose and water were mixed and boiled for 2 minutes at 80°C. When the mixture had cooled to 50°C, the mustard and egg were added, and progressively the oil, with the Silverson fitted with a square-holed grid which causes a high shearing at 4000 rpm. Then, vinegar was added. The mix was left in the Silverson for 30 seconds at 5000 rpm. Finally, the mayonnaise was packaged and cooled to 4°C.

### b) Evaluation of emulsion quality.

### Determination of the size of lipid globules using a microscope

The smaller the lipid globules, the easier the emulsification.

### Evaluation of stress required to destabilise the mayonnaise

Destabilisation of the emulsion was caused by the combined effects of vibrations (provided by a Vortex mixer) and centrifugal forces (2000 to 15000g). These parameters were measured after 1 day and 1 month in order to test the long-term stability of the emulsion. If the emulsion was stable, the period of vortex was longer and the centrifuge forces were higher than if the emulsion was less stable.

### Temperature effects

Samples were incubated at 40°C for 48 hours. Then, separation of lipid phase of the mayonnaise was evaluated.

### Rheology

Samples are analysed in constraint oscillation, with a constraint rheometer of AR500-TA instrument type, fixed with the following parameters : constraint oscillation : 0.05 to 90 Pa, 1Hz, 20°C, acrylic cone of 6 cm, 2°, "entrefrer" 50µm. Resulted curves allow to determine constraint necessary to the deconstruction of the mayonnaise texture.

### Sensory evaluation

The taste and the difference in flavour of the mayonnaises were estimated. Rheological and sensory tests demonstrate the positive effect of flour high in puroindolines in light mayonnaise texture.

### References

An et al. (1986), *Plant Physiology,* 81 :86-91
Anderson O.D. et al. (1989), TAG, 77 : 86-91
Allison et al. (1986); the MDMV leader (Maize Dwarf Mozaic Virus), *Virology,* 154 :9-20
Bevan et al. (1983), *Nature,* 304 :184-187
Callis et al. (1987), *Genes Dev.,* 1 :1183
Carrington and Freed (1990), J. virol. 64(4) :1590-1597
Christensen et al. (1996), Transgenic. Res., 5 :213
Datla, R et al. (1997), *Biotechnology Ann. Rev.,* 3 :269-296
Dekeyser et al. (1988), *Plant Physiology*, 90 :217-223
Della-Cioppa et al. (1987), *Plant Physiology,* 84 :965-968
Depicker et al. (1992), *Mol. Gen. Genet.,* 235(2-3) :389-396
Depigny-This et al. (1992), *Plant Molecular Biology*, 20 :467-479
Dubreil L. et al (1998), Effect of puroindolines on the breadmaking properties of wheat flour, *Cereal Chemistry* 75(2) :222-229
Edwards *et al.* (1991), *Nucleic Acids Research*, Vol. 19 No. 6 p. 1349
Eichholtz et al. (1987), *Somatic Cell and Molecular Genetics,* 13 :67-76
Elroy-Stein, O., Fuerest, T.R., and Moss B. (1989), *PNAS USA,* 86 :6126-6130
Fraley et al. (1983), *Proc. Natl. Acad. Sci. USA* 80 :4803
Franck et al. (1980), *Cell,* 21(1) :285-94
Fromm et al.(1985), "Expression of Genes Transferred into Monocot and Dicot Plant Cells by Electroporation", *Proc. Natl Acad*. *Sci. USA* 82 :5824
Gallie, D.R. et al. (1989), *Molecular Biology of RNA,* pages 237-256
Gaubier et al. (1993), *Molecular and General Genetics,* 238 :409-418
Gautier M-F et al (1994), *Triticum aestivum* puroindolines, two basic cystine-rich seed proteins: cDNA sequence analysis and developmental gene expression, *Plant*
*Molecular Biology,* 25 : 43-57
Giroux M.J. and Morris C.F.(1998), Wheat grain hardness results from highly conserved mutations in the friabilin components puroindoline a and b, *Proc. Natl. Acad. Sci. USA,* 95(11) pp 6262-6266
Gritz et al. (1983), *Gene,* 25 :179-188
Halford, N. *et al.* (1989), Functional analysis of the upstream regions of a silent and a expressed member of a family of wheat seed protein genes in transgenic tobacco, *Plant Science,* 62 :207-216
Hauptmann et al. (1988), *Plant Physiology,* 86 :602-606
Herrera-Estrella et al. (1983), *EMBO Journal* 2 :987-995
Hohn et al. (1982), "Molecular Biology of Plant Tumors", Academic Press, New York, pp.549-560
Horsch et al. (1984), "Inheritance of Functional Foreign Genes in Plants", *Science,* 233 :496-498
Howell, United States Patent No. 4,407,956
Jobling, S.A., and Gehrke, L. (1987), *Nature,* 325 :622-625
Jouanin et al. (1987), *Plant Science,* 53 :53-63
Kay et al. (1987), *Science*, 236 :1299-1302
Lommel, S.A. et al. (1991), *Virology,* 81 :382-385
Maas et al. (1991), *Plant Molecular Biology,* 16 :199
Macejack, D.G., and P. Sarnow (1991), *Nature,* 353 :90-94
McCormick et al. (1986), *Plant Cell Reports,* 5 :81-84
McCreery and Helentjaris (1994a), *Methods in Molecular Biology,* Vol. 28 : Protocols for nucleic acid analysis by non-radioactive probes, pp67-71, Edited by P. G. Isaac, Humana Press Inc., Totawa, NJ
McCreery and Helentjaris (1994b), *Methods in Molecular Biology,* Vol. 28 : Protocols for nucleic acid analysis by non-radioactive probes, pp107-112, Edited by P. G. Isaac, Humana Press Inc., Totawa, NJ
McElroy et al. (1990), *Plant Cell,* 2 :163-171
McElroy et al. (1991), *Molecular and General Genetics,* 231 :150-160
Meijer et al. (1991), *Plant Molecular Biology*, 16 :807-820
Morris et al. (1992), *Virology,* 187 :633
Mullis, KB (1987), *Methods in Enzymology* 155 :335
Ohta et al. (1990), *Plant Cell Physiology,* 31 :805
Paszkowski et al. (1984), *EMBO Journal* 3 :2717-22
Roberts et al. (1989), *Plant cell,* 1 :569-578
Sambrook et al. (1989), *Molecular Cloning, A Laboratory Manual, Cold Spring Harbour Laboratory Press* p. 9.54-9.62
Shen et al. (1994), *Plant Molecular Biology,* 26 :1085-1101
Snowdon et al. (1996), *Plant Molecular Biology,* 31 :689
Southern, EM (1975), *Journal of Molecular Biology,* 98 :503
Stacey and Isaac (1994), *Methods in Molecular Biology,* Vol. 28 : Protocols for nucleic acid analysis by nonradioactive probes, pp9-15, Edited by P. G. Isaac, Humana Press Inc., Totawa, NJ
Vancanneyt et al. (1990), *Molecular and General Genetics,* 220 :245-250
Waldron et al. (1985), *Plant Molecular Biology* 5 :103-108
White et al. (1990), NAR 18 :1062
*Methods in Enzymology* Vol. 153 ("Recombinant DNA Part D") 1987, Wu and Grossman Eds., Academic Press

## Claims

1. New method for increasing expression of a puroindoline protein in a plant, said puroindoline protein modifying grain hardness, notably breadmaking quality, said method comprising the steps of:
(e) preparing a DNA construct which will allow the expression of a puroindoline protein,
(f) introducing said DNA construct into plant cell, plant tissue, or plant,
(g) selecting the plant cell, or plant tissue, or plant which stably maintain the puroindoline expression,
(h) and optionally regenerating fertile stable transformed plant.

2. A method of claim 1, wherein said DNA construct is an expression cassette comprising a DNA of interest included between a promoter and a terminator.

3. A method of claim 2, wherein said promoter is the Hight Molecular Weight Glutenin of wheat.

4. A method of claim 2 or 3, wherein said DNA of interest is the puroindoline-a cDNA

5. A method of claim 2 or 3, wherein said DNA of interest is the puroindoline-b cDNA.

6. A vector comprising an expression cassette according to claim 4

7. A vector comprising an expression cassette according to claim 5

8. A host cell comprising a vector according to claim 6,7 or a combination of said vectors

9. A plant cell transformed by a vector according to claim 6,7 or a combination of said vectors

10. A transgenic plant and progeny thereof wherein said plant is regenerated from a plant cell according to claim 8.

11. The plant and progeny of claim 10 wherein the plant is chosen in the group consisting of wheat, maize, potato, barley and rice.

12. A transgenic plant transformed with an expression cassette according to claim 4 or 5, so that the puroindoline a or puroindoline b is expressed in the transgenic plant, said plant exhibiting a modified grain hardness compared to a progenitor plant which does not contain the expression cassette when the transgenic plant and the progenitor plant are cultivated under the same conditions.

13. A seed, a progeny, a clone, cell line or cell of the transgenic plant of claim 12

14. Use of a transgenic plant according to claim 8, or a part of it or a product of it, in order to prepare alimentary products or ingredients.

15. 15 Use according to claim 14 in order to prepare flour.

16. Alimentary products or ingredients obtainable according to claims 14 or 15.
